# EUROPEAN PATENT APPLICATION

(11) **EP 4 559 510 A1**
(43) Date of publication of application: **28.05.2025**
(21) Application number: 22949672.4
(22) Date of filing: 16.12.2022
(51) Int. Cl.: A61N 1/36, A61N 1/04, A61N 1/05

(54) **GALVANIC TASTE MODULATION DEVICE AND GALVANIC TASTE TESTING DEVICE**

(30) Priority: 22.07.2022 JP 2022117501
(71) Applicant: Ubeing Inc., Nagoya-shi, Aichi 453-6111 (JP)
(72) Inventor: FUKUSHIMA, Taiki, Nagoya-shi, Aichi 453-6111 (JP)
(74) Representative: Gulde & Partner
(86) International application number: PCT/JP2022/046512
(87) International publication number: WO 2024/018649

(57) **Abstract**

In an electric taste adjustment device that adjusts taste using electrical signals when eating or drinking, electrodes must be attached in order to apply electrical signals. An electric taste adjustment device is provided to eliminate this hassle. The electric taste adjustment device 1 is attached to a food container, and when the food container is taken into the mouth, the electrodes 1 and 2 connected to the electric taste adjustment device 1 contact an upper mandible and a mandibular base, respectively, to apply an electric signal. This makes it easy to attach electrodes for taste adjustment.

## Description

### Technical Field

The present invention relates to an electric taste adjustment device and an electric taste testing device that presents and regulates configured to present and adjust taste through electrical stimulation.

### Background Art

Excessive intake of salt and sugar is reported as one of the causes of health damage. On the other hand, a low-salt food that contain less salt is recommended, but it generally reduces the feeling of satisfaction with food.

As a way to present enough taste to satisfy even if it is a low-salt food that is considered to be "bland" that reduces the feeling of satisfaction with meals, a method of presenting taste by applying electrical stimulation to the taste organ in the oral cavity is disclosed. For example, Patent Literature 1 discloses a taste electric stimulation device where an electrode that apply an electrical signal is attached to the head. Patent Literature 2 discloses an electric taste presentation device where electrodes are installed on the fingertips and an electrical signal is applied through conductive tableware such as chopsticks and forks and foodstuffs.

In addition, there is an increasing demand for testing devices that can quickly and easily detect changes in taste, which is one of the early symptoms of infectious diseases such as the coronavirus, by self-checking at home. For example, there is also an increasing demand for a form that uses an electric taste adjustment device as a taste testing device, such as checking taste changes due to diseases such as coronavirus infection or malignant tumors at the early stage of infection or continuously checking taste changes due to the aftereffects of coronavirus infection. Furthermore, expectations are rising for its use in ways such as adjusting taste and enriching meals to compensate for taste disturbances caused by the after-effects of coronavirus infection.

### Citation List

### Patent Literature

[Patent Literature 1] JP-A-2018-42991
[Patent Literature 2] JP-A-2021-45399

### Summary of Invention

### Technical Problem

However, the taste electrical stimulation device of Patent Literature 1 requires electrodes to be attached to the head during the meal period, and requires work such as attaching and removing the electrodes before and after meals. In addition, wiring is required to apply electrical signals between the electrodes, and the electrodes must be held in the mouth. Therefore, these hassles become a factor that reduces meal satisfaction.

The electric taste presentation device of Patent Literature 2 attempts to eliminate the hassle of wiring by installing electrodes on fingertips and applying electrical signals through conductive tableware such as chopsticks and forks and foodstuffs. However, the hassle of having to wear gloves or the like with electrodes attached and continuing to wear the electrodes during the meal period cannot be completely eliminated.

In addition, there is a demand for a simple and hassle-free device for measuring (testing) taste disorders caused by diseases such as coronavirus infections and malignant tumors, especially for purposes such as self-confirmation at home and continuous taste checking.

### Solution to Problem

In order to solve the above-mentioned problems, the invention according to the present application is an electric taste adjustment device configured to adjust taste through electrical stimulation of a human body. The electric taste adjustment device comprises a first electrode, a second electrode and an electrical signal generator. The first electrode is configured to come into contact with an oral cavity of the human body. The second electrode is configured to come into contact with either or all of a mandibular base and a front of a neck of the human body. The electrical signal generator is configured to apply an electrical signal between the first electrode and the second electrode. And the electric taste adjustment device is configured to present taste to the human body by applying the electrical signal to a taste organ of the human body using the first electrode and the second electrode.

In addition, an electric taste adjustment device configured to adjust taste through electrical stimulation of a human body comprises a first electrode, a second electrode and an electrical signal generator. The first electrode is configured to come into contact with either or all of a mentolabial sulcus and a cheek of an upper mandible of the human body. The second electrode is configured to come into contact with either or all of a bottom of a mandibular base and a front of a neck of the human body. The electrical signal generator is configured to apply an electrical signal between the first electrode and the second electrode. And the electric taste adjustment device is configured to present taste to the human body by applying the electrical signal to a taste organ of the human body using the first electrode and the second electrode.

Further, the electric taste adjustment device according to the present invention comprises a food container holding unit configured integrally with a first electrode and a second electrode and that holds a food container. When the human body orally ingests the food in the food container held by the food container holding unit, the first electrode and the second electrode are come into contact with the human body, and the electric taste adjustment device presents taste to the human body by applying the electric signal to the taste organs of the human body.

Furthermore, the electric taste adjustment device according to the present invention includes an approach detection unit. The approach detection unit is configured to detecting approach to the human body. And the electric signal generator is configured to adjust the electric signal by the approach detection unit.

Furthermore, the electric taste adjustment device can adjust in either or all of amplitude, frequency, waveform, intermittent time of the electric signal.

In the electric taste adjustment device according to the present invention, the first electrode is an anode and the second electrode is a cathode, or the first electrode is a cathode and the second electrode is an anode.

In the electric taste adjustment device according to the present invention, it is possible to switch between the combinations of the first electrode being an anode and the second electrode being a cathode, and the first electrode being a cathode and the second electrode being an anode.

The electric taste testing device according to the present invention comprises a taste judging unit. The taste judging unit is configured to judge the taste. The electrical signal generator is configured to change the electrical signal based on a judgment information from the taste judging unit.

The electric taste testing device according to the present invention is configured to adjust taste through electrical stimulation of a human body. The electric taste testing device comprises a first electrode, a second electrode and an electrical signal generator. The first electrode is configured to come into contact with an oral cavity of the human body. The second electrode is configured to come into contact with either or all of a bottom of a mandibular base and a front of a neck of the human body. The electrical signal generator is configured to apply an electrical signal between the first electrode and the second electrode. The electric taste testing device is configured to present taste to the human body by applying the electrical signal to a taste organ of the human body using the first electrode and the second electrode.

The electric taste testing device according to the present invention is configured to adjust taste through electrical stimulation of a human body. The electric taste testing device comprises a first electrode, a second electrode and an electrical signal generator. The first electrode is configured to come into contact with either or all of a mentolabial sulcus and a cheek of an upper mandible of the human body. The second electrode is configured to come into contact with either or all of a mandibular base and a front of a neck of the human body. The electrical signal generator is configured to apply an electrical signal between the first electrode and the second electrode. The electric taste testing device is configured to present taste to the human body by applying the electrical signal to a taste organ of the human body using the first electrode and the second electrode.

According to the present invention, it is possible to perform taste presentation by contacting the electrode for applying electrical stimulation to the taste organ only when the food is in the mouth, and it is possible to present without wearing the electrode during the meal period.

### Brief Description of Drawings

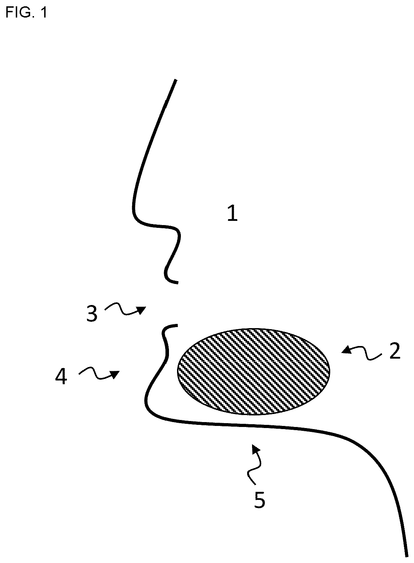

FIG. 1 is a schematic diagram of a human body where an electric taste adjustment device according to the present invention presents taste sensations.
FIG. 2 is a schematic diagram showing the positions of electrodes installed when the electric taste adjustment device according to the present invention presents taste.
FIG. 3 is a schematic diagram of a ring electrode of the electric taste adjustment device according to the present invention.
FIG. 4 is an example of a method for attaching the ring electrode of the electric taste adjustment device according to the present invention.
FIG. 5 is an example of an electric taste adjustment device including a food container holding unit according to the present invention.
FIG. 6 is a schematic diagram showing an example of use of the electric taste adjustment device including the food container holding unit according to the present invention.
FIG. 7 is a schematic diagram showing an example of use of the electric taste adjustment device including the food container holding unit according to the present invention.

### Description of Embodiments

Embodiments of the present invention will be described below with reference to the drawings. It is noted that the embodiments are not limited to the configuration and means shown in the figures.

Conventionally, a method for presenting taste through electrical stimulation of taste organs in the tongue and oral cavity is disclosed. In order to apply this electrical stimulation, various methods for installing electrodes are proposed.

For example, in Patent Literature 1 (JP-A 2018-42991), an invention where one electrode is installed at the back of the head and the back of the neck, the other electrode is installed around the oral cavity and jaw, a closed circuit in the human body is formed and an electrical signal is applied is disclosed.

For example, in Patent Literature 2 (JP-A 2018-42991), an invention where one electrode is installed on the hand holding tableware, the other electrode is installed on the tableware such as a cup or chopsticks used when eating and drinking, a closed circuit is formed between the hand, via the arm, and the tableware that comes into contact with the inside of the mouth, and an electric signal is applied is disclosed.

In contrast, the present invention configures a closed circuit around the mandible by installing one electrode in the mentolabial sulcus that is the upper mandible and the other electrode at the mandibular base, and applies electrical signals to the taste organs in the mouth.

According to this electrode arrangement, the path for applying the electric signal can be made relatively short, so it is possible to get advantages such as being able to reduce the applied voltage and suppressing the conduction of electricity to areas other than the taste organs.

Furthermore, the present invention configures a closed circuit around the mandible by installing one electrode in the oral cavity, for example, on the tongue, and the other electrode on the mandibular base, and applying electrical signals to the taste organs in the mouth. According to this electrode arrangement, the path for applying the electric signal can be made relatively short, so it is possible to get advantages such as being able to reduce the applied voltage and suppressing the conduction of electricity to areas other than the taste organs and performing more fine-grained taste adjustment by applying local electrical signals to the tongue.

Further, as an example of installing electrodes around the mandible according to the present invention, it is also possible to install one electrode on the cheek. Furthermore, as an example of installing electrodes around the mandible, it is also possible to install one electrode on the front surface of the neck. In this case, for example, by installing one electrode on the cheek and the other electrode on the mandibular base, a closed circuit can be configured to apply electrical signals to the taste organs in the mouth. Furthermore, by installing one electrode in the mentolabial sulcus and the other electrode in the front surface of the neck, a closed circuit can be configured to apply electrical signals to the taste organs in the mouth. In addition, by installing one electrode on the cheek and the other electrode on the front surface of the neck, a closed circuit can be configured to apply electrical signals to the taste organs in the mouth.

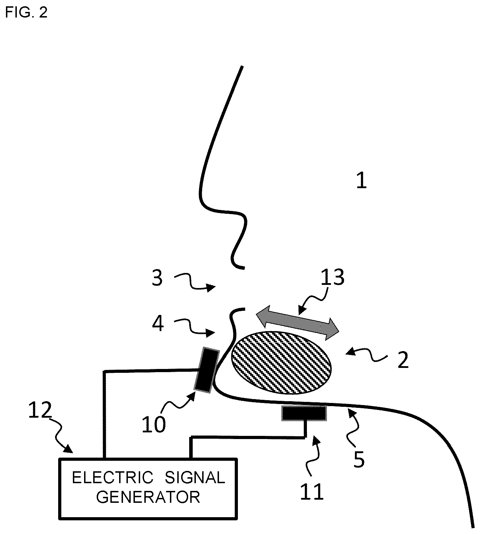

FIG. 2 shows an example where one electrode 10 is installed in the mentolabial sulcus 4 and the other electrode 11 is installed in the mandibular base 5. The electrical signal applied between the electrodes 10 and 11 stimulates the taste organ 2 to induce or suppress taste, thereby presenting the taste. An electrical signal generator (not shown) generates electrical signals with various waveforms combining the amplitude, frequency, waveform, intermittent time, etc. of the electrical signals in order to induce or suppress taste.

The electrical signal generator can enhance or suppress salty, sour, bitter, sweet, and umami by variously combining the amplitude, frequency, waveform, intermittent time, etc. of the electrical signals. Furthermore, it is possible to present taste by individually enhancing or suppressing each of salty, sour, bitter, sweet, and umami.

In addition, the electrical signal generator enhances or suppresses astringency, spiciness, fattiness, harshness, coolness, country and richness by variously combining the amplitude, frequency, waveform, intermittent time, etc. of the electrical signal. The electric signal generator can further provide the "deliciousness" obtained from the meal by presenting the spread and duration of these tastes.

FIG. 2 is a diagram showing an example where an electric signal from the electric signal generator 12 is applied via the electrodes 10 and 11. The electrical signals applied by the electrodes 10 and 11 cause a taste adjustment current 13 to flow through the taste organ 2 in the oral cavity. By flowing this taste adjustment current 13, it is possible to enhance, suppress, or present the taste in the oral cavity.

As an example of taste enhancement, suppression, or presentation, when performing anodal electrical stimulation using the electrode 10 of the mentolabial sulcus 4 in FIG. 2 as the anode and the electrode 11 of the mandibular base 5 as the cathode, it is possible to enhance salty taste etc. by various combinations of the amplitude, frequency, waveform, intermittent time, etc. of the electrical signals.

As another example of taste enhancement, suppression, or presentation, when performing cathodic electrical stimulation using the electrode 10 of the mentolabial sulcus 4 in FIG. 2 as a cathode and the electrode 11 of the mandibular base 5 as an anode, it is possible to induce a taste suppression effect by various combinations of the amplitude, frequency, waveform, intermittent time, etc. of the electrical signals, and induce a taste enhancement effect that occurs after the electrical signal is stopped.

As another example of taste enhancement, suppression, or presentation, while appropriately replacing the anodal electrical stimulation using the electrode 10 of the mentolabial sulcus 4 in FIG. 2 as an anode and the electrode 11 of the mandibular base 5 as a cathode and the cathodic electrical stimulation using the electrode 10 as the cathode and the electrode 11 as the anode, furthermore, by various combinations of the amplitude, frequency, waveform, intermittent time, etc. of the electrical signals, it is possible to induce a taste suppression effect, a taste enhancement effect that occurs after the electrical signal is stopped, and a taste suppression effect.

### [Example 1]

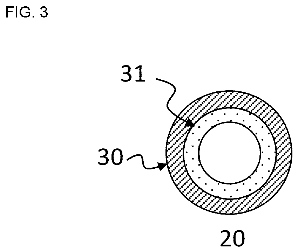

A first example of the present invention will be described. FIG. 3 shows an example where electrodes for applying electrical signals are configured in a ring shape. The ring electrode 20 shown in FIG. 3 is a view seen from the direction where a finger is inserted into a ring shape. The ring-shaped electrode 20 includes an anode or a cathode electrode 30 for applying an electric signal, an insulator 31 that ensures insulation between the electrode 30 and the hand 6 and fingers 7 to 8 when the ring-shaped electrode 20 is attached to the finger. Further, the electrode 30 is electrically connected to an electric signal generator (not shown).

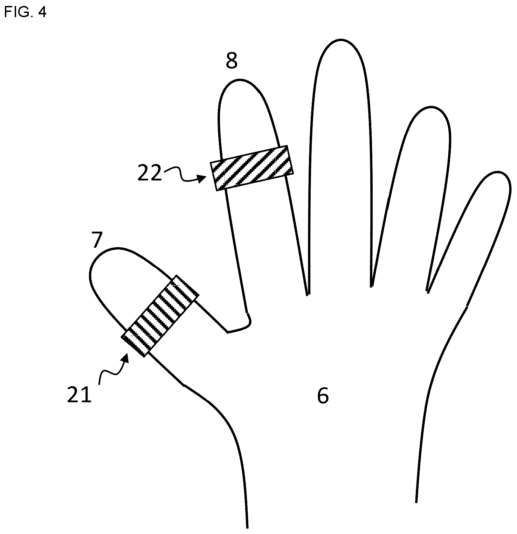

FIG. 4 shows an example where the ring electrode 21 and the other ring electrode 22 forming a pair are attached to the thumb 7 and index finger 8, respectively. However, FIG. 4 shows an example, and the fingers may be combined with any fingers of each or may be the same finger. When the electrode is attached to the finger, the insulator 31 maintains a state where no electrical signal is applied to the human body.

In the method for applying electrical signals using the electrodes 21 and 22, the thumb 7 attached with the ring electrode 21 is placed on the mandibular base 5, and at the same time, the index finger 8 attached with the ring electrode 22 is placed on the mentolabial sulcus 4, and the thumb 7 and the index finger 8 are held so as to sandwich the mandible. At this time, the electrode 21 and the electrode 22 are come into contact with the mandibular base 5 and the mentolabial sulcus 4, respectively, it is possible to apply an electric signal to the taste organ 2.

As described above, according to the first example of the present invention, it is possible to apply an electrical signal by a simple action of pinching the mandible between two fingers at any desired timing to adjust the taste when eating or drinking. Since the ring electrode only needs to be attached to one hand 6, it is possible to eliminate the hassle of having to attach the electrode around the head during the meal period.

### [Example 2]

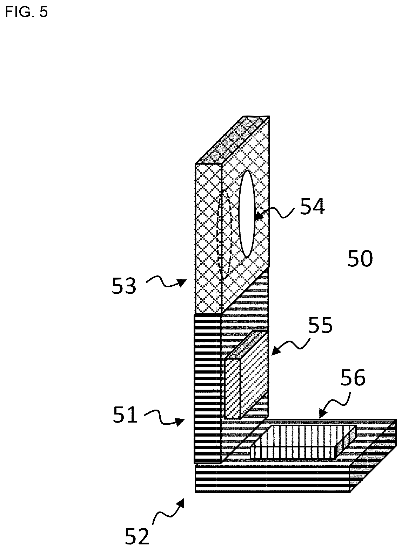

Next, a second example of the present invention will be described. FIG. 5 is a schematic diagram of an electric taste adjustment device 50 where a food container holding unit 53 and two electrodes are integrated. The electrode 55 and the electrode 56 are fixed to the electrode holding unit 51 and the electrode holding unit 52, respectively. The electrode holding unit 51 and the electrode holding unit 52 may be configured as one unit or may be configured separately, and there is no particular restriction on the configuration means. However, as will be described later, it is desirable that the shape is such that the electrode 55 and the mentolabial sulcus 4 are come in contact with each other, and at the same time, the electrode 56 and the mandibular base 5 are come in contact with each other.

The electrode holding unit 51 or the electrode holding unit 52 is configured integrally with the food container holding unit 53. However, each unit may be connected via a movable unit, or may be configured integrally using a flexible material (for example, plastic or rubber) as a base. Furthermore, the electrodes 55 and 56 are electrically connected to an electrical signal generator (not shown).

A hole 54 for holding and fixing the drinking spout of the food container is provided in the food container holding unit 53. The drinking spout of the food container is penetrated by the hole 54, and the entire food container is held by tightening, pinching, or closely contacting the drinking spout, and the electric taste adjustment device 50 is held in the food container.

Examples of food containers held by the food container holding unit 53 include plastic bottles, pouch containers, bowls, and suckers.

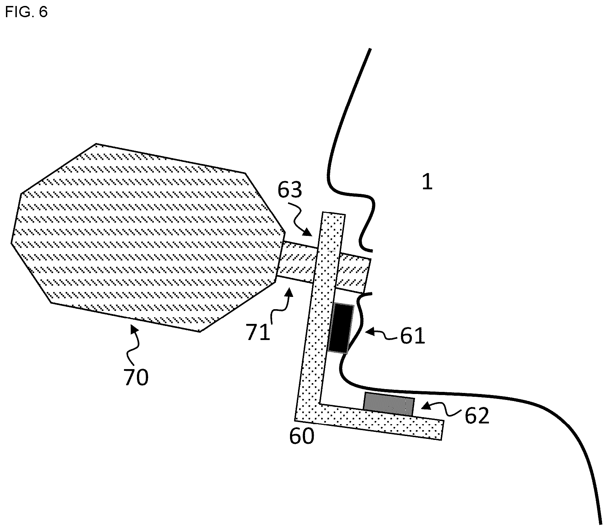

FIG. 6 is a schematic diagram when the drinking spout 71 of the food container 70 is held in the food container holding unit 63 and the food inside the food container 70 is ingested from the drinking spout 71. At this time, the electrodes 61 and 62 are come into contact with the mentolabial sulcus 4 and the mandibular base 5, respectively, and a closed circuit where electrical signals can be applied to adjust taste is configured. An electric signal for adjusting the taste is applied by an electric signal generator (not shown).

When the drinking spout 71 is removed from the mouth, the electrodes 61 and 62 are separated from the mentolabial sulcus 4 and the mandibular base 5, respectively, so that the application of electrical signals is also stopped. In addition to applying and stopping the electric signal by contacting and non-contacting the electrodes, the distance between the electrode and the human body may be measured using a proximity sensor or a contact sensor (not shown) to apply and stop the electric signal.

As described above, according to the electric taste adjustment device of Example 2 according to the present invention, since the electrodes are installed only when taking food or drink from the food container, the hassle of having to wear electrodes during meals is eliminated.

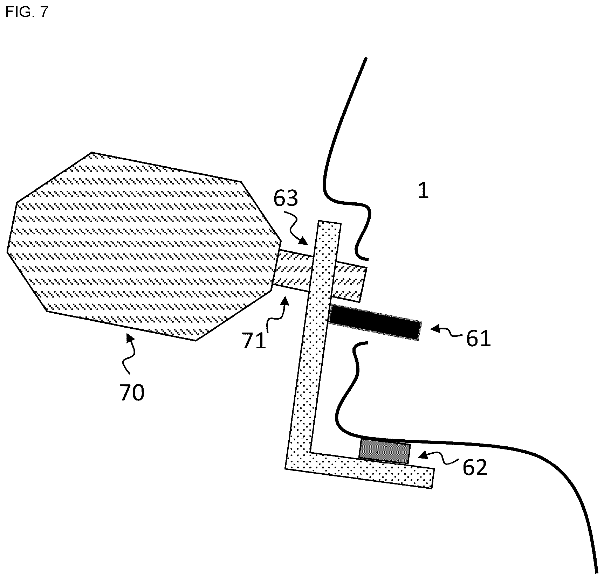

FIG. 7 shows an example where the electrode 61 of Example 2 is configured to be come into contact with the inside of the oral cavity. When the drinking spout 71 of the food container 70 is held in the food container holding unit 63 and the food in the food container 70 is ingested from the drinking spout 71, the electrode 61 is inserted into the oral cavity and come into contact with the taste organ (for example, the tongue), the electrode 62 is come into contact with the mandibular base 5 and a closed circuit capable of applying an electrical signal for adjusting taste is configured. An electric signal for adjusting the taste is applied by an electric signal generator (not shown).

When the drinking spout 71 is removed from the mouth, the electrode 61 is withdrawn from the oral cavity and the electrode 62 is separated from the mandibular base 5, so that application of the electrical signal is also stopped. In addition to applying and stopping the electric signal by contacting and non-contacting the electrodes, the distance between the electrode and the human body may be measured using a proximity sensor or a contact sensor (not shown) to apply and stop the electric signal.

### [Example 3]

The electrode used in the electric taste adjustment device according to the present invention is not limited to metal electrode, and may be conductive such as conductive plastic. For example, by integrally molding the electrode with conductive plastic and molding it to follow the shape of the mandible, the adhesion of the electrode can be increased.

### [Example 4]

The electric taste adjustment device described so far enhances, decreases, or induces the presentation of taste when eating or drinking by applying an electric current through the taste organs of the human body, but it is also possible to present or induce the taste even when there is no food or drink in the mouth. By directly utilizing this principle, the electric taste adjustment device according to the present invention can be used as an electric taste testing device (taste function measuring device) for the human body.

As an example of an electric taste testing device (taste function measuring device), an electric taste adjustment device having the configuration according to the second example described above will be described as an example. The electrodes 55 and 56 are come into contact with the mentolabial sulcus 4 and the mandibular base 5 of subject with no food or drink in their mouths, respectively. Next, the inspector applies an electric signal between the electrodes 55 and 56 using a control means (not shown). The subject judges the type and strength of taste sensed while the electrical signal is being applied, or before and after the electrical signal is applied.

It is possible to confirm the function of the subject's taste organs by comparing taste enhancement, reduction, or presentation effect estimated from the polarity, amplitude, frequency, waveform, intermittent time, etc. of the applied electrical signal, and the taste sensed by the subject, taste not sensed by the subject, enhanced taste, or decreased taste and the taste presented or evoked.

The type of taste sensed by the subject and judgment of its strength are transmitted to the electric taste adjustment device by a taste judging unit (not shown). The electric taste adjustment device continues or stops applying the electric signal based on the judgment information transmitted by the taste judging unit, and also changes the polarity, amplitude, frequency, waveform, intermittent time, etc. of the electric signal. As an example of the taste judging unit , there is an input means composed of a push button switch, a slider bar, or the like. The subject expresses the sensed taste, no sensed taste, enhanced taste, decreased taste, presented or evoked taste by means of the illustrated push button switch, slider bar.

It is possible for the electric taste adjustment device to confirm the subject's taste function by relatively or objectively comparing the subject's taste sensing state transmitted by the taste judging unit and the state of the applied electrical signal.

### Reference Signs List

1: subject,
2: taste organ,
3: mouth,
4: mentolabial sulcus,
5: mandibular base,
6: hand,
7: thumb,
8: index finger,
10: electrode,
11: electrode,
12: electric signal generator,
13: taste adjustment current,
20: ring electrode,
21: ring electrode attached to finger,
22: ring electrode attached to finger,
30: electrode part,
31: insulator,
50: electric taste adjustment device,
51: electrode holding unit,
52: electrode holding unit,
53: food container holding unit,
54: hole for holding food container,
55: electrode,
56: electrode,
60: electric taste adjustment device,
61: electrode,
62: electrode,
63: hole for holding food container,
70: food container,
71: drinking spout of food container

## Claims

1. An electric taste adjustment device configured to adjust taste through electrical stimulation of a human body, comprising:
a first electrode;
a second electrode; and
an electrical signal generator, wherein
the first electrode is configured to come into contact with an oral cavity of the human body,
the second electrode is configured to come into contact with either or all of a mandibular base and a front of a neck of the human body,
the electrical signal generator is configured to apply an electrical signal between the first electrode and the second electrode, and
the electric taste adjustment device is configured to present taste to the human body by applying the electrical signal to a taste organ of the human body using the first electrode and the second electrode.

2. An electric taste adjustment device configured to adjust taste through electrical stimulation of a human body, comprising:
a first electrode;
a second electrode; and
an electrical signal generator, wherein
the first electrode is configured to come into contact with either or all of a mentolabial sulcus and a cheek of an upper mandible of the human body,
the second electrode is configured to come into contact with either or all of a mandibular base and a front of a neck of the human body,
the electrical signal generator is configured to apply an electrical signal between the first electrode and the second electrode, and
the electric taste adjustment device is configured to present taste to the human body by applying the electrical signal to a taste organ of the human body using the first electrode and the second electrode.

3. An electric taste adjustment device configured to adjust taste through electrical stimulation of a human body, comprising:
a first electrode;
a second electrode;
an electrical signal generator; and
a food container holding unit, wherein
the first electrode is configured to come into contact with either or all of a mentolabial sulcus and a cheek of an upper mandible of the human body,
the second electrode is configured to come into contact with either or all of a mandibular base and a front of a neck of the human body,
the electrical signal generator is configured to apply an electrical signal between the first electrode and the second electrode,
the food container holding unit is configured to hold a food container, and
the electric taste adjustment device is configured to present taste to the human body by applying the electrical signal to a taste organ of the human body using the first electrode and the second electrode where the food container holding unit is integrally configured.

4. An electric taste adjustment device configured to adjust taste through electrical stimulation of a human body, comprising:
a first electrode;
a second electrode;
an electrical signal generator; and
a food container holding unit, wherein
the first electrode is configured to come into contact with either or all of a mentolabial sulcus and a cheek of an upper mandible of the human body,
the second electrode is configured to come into contact with either or all of a mandibular base and a front of a neck of the human body,
the electrical signal generator is configured to apply an electrical signal between the first electrode and the second electrode,
the food container holding unit is configured to hold a food container,
the first electrode, the second electrode, and the food container holding unit are integrally configured, and
the electric taste adjustment device is configured to present taste to the human body by applying the electrical signal to a taste organ of the human body when the first electrode and the second electrode come into contact with the human body during an action of eating and drinking food in the food container held by the food container holding unit.

5. An electric taste adjustment device of any one of claims 1 to 4, wherein
the electric taste adjustment device includes an approach detection unit,
the approach detection unit is configured to detecting approach to the human body, and
the electric signal generator is configured to adjust the electric signal by the approach detection unit.

6. An electric taste adjustment device of any one of claims 1 to 4, wherein
the electrical signal is adjustable in either or all of amplitude, frequency, waveform, intermittent time.

7. An electric taste adjustment device of any one of claims 1 to 4, wherein
the first electrode is an anode, and
the second electrode is a cathode.

8. An electric taste adjustment device of any one of claims 1 to 4, wherein
the first electrode is a cathode, and
the second electrode is an anode.

9. An electric taste adjustment device of any one of claims 1 to 4, wherein
the electrical signal is adjustable in either or all of amplitude, frequency, waveform, intermittent time, and
the electrical signal is switchable between an anodic electrical stimulation state where the first electrode is an anode and the second electrode is a cathode, and a cathodic electrical stimulation state where the first electrode is a cathode and the second electrode is an anode.

10. An electric taste testing device configured to adjust taste through electrical stimulation of a human body, comprising:
a first electrode;
a second electrode; and
an electrical signal generator; and
a taste judging unit, wherein
the first electrode is configured to come into contact with an oral cavity of the human body,
the second electrode is configured to come into contact with either or all of a mandibular base and a front of a neck of the human body,
the electrical signal generator is configured to apply an electrical signal between the first electrode and the second electrode,
the electric taste testing device is configured to present taste to the human body by applying the electrical signal to a taste organ of the human body using the first electrode and the second electrode,
the taste judging unit is configured to judge the taste presented, and
the electrical signal generator is configured to change the electrical signal based on a judgment information from the taste judging unit.

11. An electric taste testing device configured to adjust taste through electrical stimulation of a human body, comprising:
a first electrode;
a second electrode; and
an electrical signal generator; and
a taste judging unit, wherein
the first electrode is configured to come into contact with either or all of a mentolabial sulcus and a cheek of an upper mandible of the human body,
the second electrode is configured to come into contact with either or all of a mandibular base and a front of a neck of the human body,
the electrical signal generator is configured to apply an electrical signal between the first electrode and the second electrode, and
the electric taste testing device is configured to present taste to the human body by applying the electrical signal to a taste organ of the human body using the first electrode and the second electrode,
the taste judging unit is configured to judge the taste presented, and
the electrical signal generator is configured to change the electrical signal based on a judgment information from the taste judging unit.
